(19) **Europäisches Patentamt / European Patent Office / Office européen des brevets**

(11) **EP 4 725 935 A1**

(12) **EUROPEAN PATENT APPLICATION**
published in accordance with Art. 153(4) EPC

(43) Date of publication:
15.04.2026 Bulletin 2026/16

(21) Application number: 24819276.7

(22) Date of filing: 31.05.2024

(51) International Patent Classification (IPC):
$C07C\ 68/08^{(2006.01)}$    $C07B\ 61/00^{(2006.01)}$
$C07C\ 68/06^{(2020.01)}$    $C07C\ 69/96^{(2006.01)}$

(52) Cooperative Patent Classification (CPC):
(C-Sets available)
C07C 68/06; C07B 61/00; C07C 68/08    (Cont.)

(86) International application number:
PCT/JP2024/020116

(87) International publication number:
WO 2024/253046 (12.12.2024 Gazette 2024/50)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 08.06.2023 JP 2023094944

(71) Applicant: Asahi Kasei Kabushiki Kaisha
Tokyo 100-0006 (JP)

(72) Inventor: ENOMOTO, Miyako
Tokyo 100-0006 (JP)

(74) Representative: Strehl & Partner mbB
Maximilianstrasse 54
80538 München (DE)

(54) **METHOD FOR PRODUCING CARBONATE HAVING ETHYL GROUP AND APPARATUS FOR PRODUCING CARBONATE HAVING ETHYL GROUP**

(57) A method for producing a carbonate having an ethyl group, the method comprising a reaction step of subjecting dimethyl carbonate and ethanol to transesterification reaction in a reactor in the presence of a catalyst containing an alkali metal compound to obtain a reaction composition, a solvent addition step of adding a solvent to the reaction composition, and a distillation step of distilling the reaction composition in a distillation column.

[Figure 1]

(52) Cooperative Patent Classification (CPC): (Cont.)

C-Sets
**C07C 68/06, C07C 69/96;**
**C07C 68/08, C07C 69/96**

## Description

### Technical Field

The present invention relates to a method for producing a carbonate having an ethyl group and an apparatus for producing a carbonate having an ethyl group.

### Background Art

Carbonates having an ethyl group, such as ethyl methyl carbonate (EMC) and diethyl carbonate (DEC), are used as the organic solvent for battery electrolyte solutions. These carbonate compounds are known to be obtained from the transesterification reaction of dimethyl carbonate (DMC) and ethanol (EtOH). In the aforementioned transesterification reaction, an alkali metal compound such as an alkoxide compound of an alkali metal is used as the catalyst because it exhibits excellent activity (see Patent Literature 1 and Patent Literature 2).

### Citation List

### Patent Literature

Patent Literature 1: International Publication No. WO 2022/114592

Patent Literature 2: International Publication No. WO 2022/114576

### Summary of Invention

### Technical Problem

Upon production of a carbonate having an ethyl group, an alkali metal compound is used as the catalyst for transesterification reaction. As the catalyst, sodium methoxide or sodium hydroxide (hereinafter, also referred to as "NaOH") is used.

However, the alkali metal compound has a low solubility in an organic solvent, and for example, has properties of having a low solubility in DMC, EMC, and DEC, and the precipitation of the alkali metal compound may cause process troubles.

Regarding this, Patent Literature 1 suggests a method for supplying an alkali metal compound in a state of being dissolved in dimethylsulfoxide. However, the alkali metal compound has a low solubility in dimethylsulfoxide as small as less than 1% by mass, and impurities are produced when raw materials, the alkali metal compound, and dimethylsulfoxide are mixed and heated.

Although Patent Literature 2 discloses a method for removing insoluble matter by providing a filtration apparatus in a step subsequent to the reaction step, the filtration apparatus has problems in that it requires cleaning work and maintenance work, it causes blockage in piping for supplying materials to the filtration apparatus, and the like, and has further problems in that solid wastes derived from catalysts are strongly alkaline and water prohibitive, have high risks, and are hardly disposed.

An object of the present invention is to provide a method for producing a carbonate having an ethyl group in which the production of a precipitate can be suppressed in the distillation step and the distillation step can be continuously operated for a long term, and an apparatus for producing a carbonate having an ethyl group.

### Solution to Problem

The present inventors have found that the aforementioned problems can be solved by setting a reaction composition immediately before introduction into the distillation column in the distillation step to have a concentration of insoluble matter having a size of 10 $\mu$m or more in the reaction composition of a predetermined value or less.

The present invention encompasses the following embodiments.

<1> A method for producing a carbonate having an ethyl group, the method comprising:

a reaction step of subjecting dimethyl carbonate and ethanol to transesterification reaction in a reactor in the presence of a catalyst containing an alkali metal compound to obtain a reaction composition;

a solvent addition step of adding a solvent to the reaction composition; and

a distillation step of distilling the reaction composition in a distillation column.

<2> A method for producing a carbonate having an ethyl group, the method comprising:

a reaction step of subjecting dimethyl carbonate and ethanol to transesterification reaction in a reactor in the presence of a catalyst containing an alkali metal compound to obtain a reaction composition; and

a distillation step of distilling the reaction composition in a distillation column,

wherein the concentration of insoluble matter having a size of 10 $\mu$m or more in the reaction composition immediately before introduction into the distillation column in the distillation step is 50 ppm by mass or less.

<3> The method for producing a carbonate having an ethyl group according to <1>, the method comprising thesolvent addition step of adding a solvent to the reaction composition before the distillation step.

<4> The method for producing a carbonate having an ethyl group according to <1> or <3>, wherein insoluble matter in the reaction composition is dissolved.

<5> The method for producing a carbonate having an ethyl group according to any one of <1> and <3> to <4>, the method comprising an acid addition step of adding an acidic substance having a pKa of 6.0 or less to the reaction composition before the solvent addition step.

<6> The method for producing a carbonate having an ethyl group according to <5>, wherein the acidic substance is at least one selected from the group consisting of carboxylic acid and sulfonic acid.

<7> The method for producing a carbonate having an ethyl group according to <6>, wherein the acidic substance is at least one selected from the group consisting of propanoic acid, butanoic acid, pentanoic acid, hexanoic acid, chloroacetic acid, dichloroacetic acid, trichloroacetic acid, and oleic acid.

<8> The method for producing a carbonate having an ethyl group according to any one of <1> and <3> to <7>, wherein each of the alkali metal compound and a reaction product of the alkali metal compound and the acidic compound has a solubility of 10% by mass or more at 30°C in the solvent.

<9> The method for producing a carbonate having an ethyl group according to any one of <1> and <3> to <8>, wherein the solvent has a higher boiling point than a boiling point of diethyl carbonate under ordinary pressure.

<10> The method for producing a carbonate having an ethyl group according to any one of <1> and <3> to <9>, wherein the solvent contains a compound having at least one hydroxyl group.

<11> The method for producing a carbonate having an ethyl group according to any one of <1> and <3> to <10>, wherein the solvent contains at least one selected from the group consisting of monoethylene glycol, diethylene glycol, triethylene glycol, 1-hexanol, 2-hexanol, 1-heptanol, and 2-heptanol.

<12> The method for producing a carbonate having an ethyl group according to any one of <1> and <3> to <10>, wherein an amount of the solvent added in the solvent addition step is 0.1 parts by mass to 20 parts by mass per 100 parts by mass of the reaction composition.

<13> The method for producing a carbonate having an ethyl group according to any one of <1> to <12>, wherein the alkali metal compound is a sodium-containing compound.

<14> The method for producing a carbonate having an ethyl group according to any one of <1> to <13>, wherein a concentration of the alkali metal compound is 1 ppm by mass to 30,000 ppm by mass based on the total amount of the reaction composition.

<15> The method for producing a carbonate having an ethyl group according to any one of <1> to <14>, wherein the reactor is a stirred reactor or a distillation column.

<16> The method for producing a carbonate having an ethyl group according to any one of <1> to <15>, wherein the distillation step is a step of separating a fraction containing 99.9% by mass or more of the carbonate having an ethyl group.

<17> The method for producing a carbonate having an ethyl group according to any one of <1> to <16>, wherein the carbonate having an ethyl group is ethyl methyl carbonate or dimethyl carbonate.

<18> An apparatus for producing a carbonate having an ethyl group, the apparatus comprising:

a reaction apparatus for subjecting dimethyl carbonate and ethanol to transesterification reaction in a reactor in the presence of a catalyst containing an alkali metal compound to obtain a reaction composition;

a solvent addition unitfor adding a solvent to the reaction composition; and

a distillation apparatus for distilling the reaction composition.

**Advantageous Effect of Invention**

The present invention can provide a method for producing a carbonate having an ethyl group in which the production of a precipitate can be suppressed in the distillation step and the distillation step can be continuously operated for a long term, and an apparatus for producing a carbonate having an ethyl group.

**Brief Description of Drawings**

[Figure 1] Figure 1 is a block diagram of the apparatus for producing a carbonate having an ethyl group according to the present embodiment.

[Figure 2] Figure 2 is a schematic configuration diagram of an apparatus A for producing a carbonate having an ethyl group according to the present embodiment.

[Figure 3] Figure 3 is a schematic configuration diagram of an apparatus B for producing a carbonate having an ethyl group according to the present embodiment.

[Figure 4] Figure 4 is a schematic configuration diagram of an apparatus C for producing a carbonate having an ethyl group according to the present embodiment.

**Description of Embodiments**

Hereinafter, the embodiment of the present invention (hereinafter, also referred to as "the present embodiment") will be described in detail with reference to the drawings as needed; however, the present invention is not limited to these embodiments, and can be variously modified without departing from the gist thereof. Unless otherwise specified, the positional relationship in the embodiment shown in the drawings, such as up, down, left, and right, is based on the positional relationship shown in the drawings. Further, the dimensional ratio in the drawings is not limited to the illustrated ratio.

The method for producing a carbonate having an ethyl group according to the present embodiment includes:

a reaction step of subjecting dimethyl carbonate and ethanol to transesterification reaction in a reactor in the presence of a catalyst containing an alkali metal compound to obtain a reaction composition;

a solvent addition step of adding a solvent to the reaction composition; and

a distillation step of distilling the reaction composition in a distillation column.

The above structure enables to provide the method for producing a carbonate having an ethyl group in which the production of a precipitate can be suppressed in the distillation step and the distillation step can be continuously operated for a long term.

Alternatively, the method for producing a carbonate having an ethyl group according to the present embodiment includes:

a reaction step of subjecting dimethyl carbonate and ethanol to transesterification reaction in a reactor in the presence of a catalyst containing an alkali metal compound to obtain a reaction composition; and

a distillation step of distilling the reaction composition in a distillation column,

wherein the concentration of insoluble matter in the reaction composition immediately before introduction into the distillation column in the distillation step is 50 ppm by mass or less.

The above structure enables to provide the method for producing a carbonate having an ethyl group in which the production of a precipitate can be suppressed in the distillation step and the distillation step can be continuously operated for a long term.

<Concentration of Insoluble Matter>

When the concentration of insoluble matter having a size of 10 $\mu$m or more in the reaction composition immediately before introduction into the distillation column is 50 ppm by mass or less, the distillation step can be continuously operated for a long term. Since a catalyst containing an alkali metal compound has a low solubility in an organic solvent such as dimethyl carbonate, insoluble matter is likely to be generated in the reaction composition. When the reaction composition is distilled in a state of containing a large amount of insoluble matter, a precipitate is generated in the distillation column, and the operation for a long term is difficult. Thus, the relationship between the precipitate in the reaction composition and the generation of the precipitate in the distillation column is examined, and as a result, it is found that the occurrence of precipitation of the alkali metal compound in the distillation column can be suppressed by setting the concentration of insoluble matter having a size of 10 $\mu$m or more in the reaction composition to 50 ppm by mass or less.

The concentration of insoluble matter having a size of 10 $\mu$m or more in the reaction composition immediately before introduction into the distillation column is preferably 30 ppm by mass or less, more preferably 10 ppm by mass or less, still more preferably 5 ppm by mass or less, and still more preferably 1 ppm by mass or less.

The method for measuring the concentration of insoluble matter in the reaction composition is the method described in Examples.

Regarding the method for adjusting the concentration of insoluble matter having a size of 10 $\mu$m or more in the reaction composition to the aforementioned range, the concentration of insoluble matter can also be suppressed low by adding a solvent in which the solubility of the alkali metal compound is high, in the reaction step described below and thereby dissolving the alkali metal compound in the reaction composition. Examples of the method for adjusting the concentration of insoluble matter having a size of 10 $\mu$m or more in the reaction composition to the aforementioned range include the step of adding a solvent described below. In addition, as a method for effectively using a solvent having a high reactivity with the reaction composition, an acidic substance may be added in the acid addition step described below for dissolving the alkali metal compound in an inactivated state in the reaction composition.

The method for producing a carbonate having an ethyl group according to the present embodiment may include a raw material supply step of supplying a raw material containing dimethyl carbonate and ethanol to the reactor before the reaction step. In addition, the method for producing a carbonate having an ethyl group according to the present embodiment may include a reaction composition extraction step of extracting the reaction composition from the reactor after the reaction step.

The method for producing a carbonate having an ethyl group according to the present embodiment may be a continuous reaction by including a raw material supply step of continuously supplying a raw material containing dimethyl carbonate and ethanol to the reactor, and a reaction composition extraction step of continuously extracting the reaction composition from the reactor. The continuous reaction causes no blockage in the reactor and enables a high conversion rate of the raw material to be achieved.

<Raw Material Supply Step>

In the raw material supply step, the raw material is supplied to the reactor. The supply of the raw material may be continuously carried out.

The raw material contains dimethyl carbonate (hereinafter, simply referred to as "DMC") and ethanol (hereinafter, also referred to as "EtOH"). The raw material may contain a catalyst containing an alkali metal compound.

Examples of the alkali metal compound used as the catalyst include sodium hydroxide, potassium hydroxide, and an alkoxide of an alkali metal. Examples of the alkoxide of an alkali metal include lithium methoxide, lithium ethoxide, sodium methoxide, sodium methoxide, sodium ethoxide, potassium methoxide, and potassium ethoxide. Among these, the alkali metal compound is preferably a sodium-containing compound, more preferably sodium methoxide or sodium ethoxide, and still more preferably sodium methoxide, because the activity is high and thus economical operation with a low catalyst concentration is possible.

The concentration of the alkali metal compound is preferably 1 ppm by mass to 30,000 ppm by mass, more preferably 10 ppm by mass to 1,000 ppm by mass, and still more preferably 25 ppm by mass to 500 ppm by mass based on the total amount of the reaction composition.

<Reaction Step>

In the reaction step, dimethyl carbonate and ethanol are subjected to transesterification reaction in a reactor in the presence of a catalyst containing an alkali metal compound to obtain a reaction composition containing a carbonate having an ethyl group. That is, the methoxy group in dimethyl carbonate is subjected to transesterification reaction with ethanol, so that a carbonate having an ethyl group is obtained. Examples of the carbonate having an ethyl group include ethyl methyl carbonate and diethyl carbonate.

(Reactor)

The reactor is not particularly limited, and may be either a stirred reactor or a reactive distillation column. In the case of using a distillation column as the reactor, the concentration of insoluble matter having a size of 10 μm or more in the reaction composition in the reactor is preferably 50 ppm by mass or less, from the viewpoint of suppressing the precipitation of the catalyst in the distillation column in the reaction step.

The reaction temperature in the reaction step is preferably 40°C to 150°C, more preferably 50°C to 130°C, and still more preferably 60°C to 120°C. Usually, to achieve a high raw material conversion rate by continuous operation, an increase in the reaction rate by an increase in the reaction temperature or an increase in the reaction time by an increase in the residence time in the reactor are considered. However, in the reaction system according to the present embodiment, it is found that the high raw material conversion rate can be surprisingly achieved by reducing the reaction temperature and reducing the residence time in the reactor, because the influence of the reduction in the catalytic activity is minimalized.

The pressure in the reaction step may be, for example, ordinary pressure to 1000 kPaG.

<Extraction step>

In the extraction step, the reaction composition is extracted from the reactor. The extraction of the reaction composition may be continuously carried out. The extraction rate of the reaction composition is preferably set to achieve the aforementioned reactor residence time.

<Acid addition step>

The method for producing a carbonate having an ethyl group according to the present embodiment may include an acid addition step of adding an acidic substance having a pKa of 6.0 or less to the reaction composition. The acid addition step can deactivate the alkali metal compound remaining in the reaction composition. The acid addition step carried out before the solvent addition step described below suppresses the production of by-products, even when a compound having a hydroxy group that may cause transesterification reaction is used as the solvent.

The pKa of the acidic substance is preferably 5.8 or less, more preferably 5.6 or less, and still more preferably 5.4 or less. The upper limit of the pKa of the acidic substance is not particularly limited, and is preferably 0.1 or more, more preferably 1.0 or more, and still more preferably 2.0 or more.

Examples of the acidic substance include organic acids. Examples of the organic acid include carboxylic acids and sulfonic acids. Examples of the carboxylic acid include carboxylic acid having 2 to 30 carbon atoms, and more specific examples thereof include acetic acid, dichloroacetic acid, propionic acid, butanoic acid, hexanoic acid, and oleic acid.

Among these acidic substances, at least one selected from the group consisting of carboxylic acids is preferable, and at least one selected from the group consisting of propanoic acid, butanoic acid, pentanoic acid, hexanoic acid, chloroacetic acid, dichloroacetic acid, trichloroacetic acid, and oleic acid is more preferable.

The amount of the acidic substance added in the acid addition step is preferably 2.0 to 15.0, more preferably 2.5 to 12.0, and still more preferably 3.0 to 10.0 in a molar ratio of the acidic substance/the catalyst.

In the acid addition step, the acidic substance may be added in a stirred tank, or the reaction composition and the acidic substance may be joined together and then mixed using a static mixer.

<Solvent addition step>

In the solvent addition step, a solvent is added to the reaction composition before the distillation step described below. The catalyst contained in the reaction composition can be dissolved in the added solvent, thus more significantly suppressing the production of the precipitate in the distillation step. The insoluble matter in the reaction composition is preferably dissolved in the solvent addition step. That is, the solvent is preferably added in such an amount that the insoluble matter contained in the reaction composition is dissolved.

Here, each of the alkali metal compound and the reaction product of the alkali metal compound and the acidic compound preferably has a solubility of 10% by mass or more at 30°C in the solvent to be added. By having the solubility, the catalyst contained in the reaction composition can further be dissolved, and the production of the precipitate in the distillation step can be more significantly suppressed.

The solubility of each of the alkali metal compound and the reaction product of the alkali metal compound and the acidic compound in the solvent is more preferably 10% by mass or more, and still more preferably 15% by mass or more at 30°C. The above solubility may be, for example, 50% by mass or less at 30°C.

From the viewpoint of easily maintaining the dissolving performance of the column bottom liquid until the end of the distillation step mentioned below, the solvent preferably has (a) a higher boiling point than the boiling point of diethyl carbonate under ordinary pressure, or (b) an azeotropic point with diethyl carbonate under ordinary pressure.

The boiling point of the solvent is preferably 136°C or more, more preferably 140°C to 400°C, and still more preferably 150°C to 300°C under ordinary pressure.

From the viewpoint of enhancing the distillation efficiency of diethyl carbonate, it is preferable that the solvent have a higher boiling point than the boiling point of diethyl carbonate and be not azeotropic with diethyl carbonate under ordinary pressure. The solvent more preferably has a boiling point 10°C or more higher than the boiling point of diethyl carbonate, still more preferably has a boiling point 20°C or more higher than the boiling point of diethyl carbonate, and still more preferably has a boiling point 30°C or more higher than the boiling point of diethyl carbonate. When the solvent has the above boiling point, the solvent is preferably not azeotropic with diethyl carbonate under ordinary pressure.

Addition of the solvent having a higher boiling point than the boiling point of diethyl carbonate and being not azeotropic with diethyl carbonate under ordinary pressure allows the solvent added to remain until the end of the distillation step in the distillation column and to be distilled out of the column bottom after the distillation step. Thus, the above solvent is preferable in terms of not requiring the separation of the carbonate having an ethyl group that is the target compound distilled out of the column top and the solvent.

In the case of the solvent having an azeotropic point with diethyl carbonate under ordinary pressure, a solvent which has a lower boiling point than the boiling point of diethyl carbonate under ordinary pressure and in which diethyl carbonate and the composition of the solvent have an azeotropic point in a high diethyl carbonate concentration and a low solvent concentration is preferable, and a solvent having no azeotropic point with dimethyl carbonate or ethyl methyl carbonate, or even when the solvent has an azeotropic point therewith, a solvent having an azeotropic point in a high carbonate concentration and a low solvent concentration is preferable. In an atmospheric distillation column in which a catalyst is removed by being dissolved in a solvent, the solvent azeotropic with diethyl carbonate is distilled out together with diethyl carbonate from the column top. However, when a solvent whose relative volatility to diethyl carbonate is significantly changed by the change in pressure is selected, separation by distillation is possible by changing the pressure after atmospheric distillation. In the case of the solvent azeotropic with diethyl carbonate under ordinary pressure, the amount of diethyl carbonate remaining in the column bottom until after the distillation step tends to be increased. From the viewpoint of the recovery rate of diethyl carbonate, a solvent having a higher boiling point than the boiling point of diethyl carbonate under ordinary pressure is preferably selected. The aforementioned high diethyl carbonate concentration means 50% by mass or more. The aforementioned high diethyl carbonate concentration is preferably 60% by mass or more, and more preferably 70% by mass or more. The aforementioned low solvent concentration means 50% by mass or less. The aforementioned low solvent concentration is preferably 40% by mass or less, and more preferably 30% by mass or less. A solvent having an azeotropic point with diethyl carbonate in the above concentration range is preferably selected.

The solvent to be added in the solvent addition step preferably contains a compound having at least one hydroxyl group, and more preferably contains a compound having at least two hydroxyl groups. Examples of the solvent include monoethylene glycol, diethylene glycol, triethylene glycol, 1-hexanol, 2-hexanol, 1-heptanol, 2-heptanol, and 1-butanol. Among these, monoethylene glycol, diethylene glycol, or triethylene glycol is preferable.

The content of the aforementioned compound having at least one hydroxyl group is preferably 50% by mass to 100% by mass, more preferably 70% by mass to 100% by mass, and still more preferably 90% by mass to 100% by mass based on the total amount of the solvent.

The amount of the solvent added in the solvent addition step is preferably 0.1 parts by mass to 20 parts by mass, more preferably 0.5 parts by mass to 15 parts by mass, and still more preferably 1.0 part by mass to 10 parts by mass per 100 parts by mass of the reaction composition, from the viewpoint of more significantly suppressing the production of the precipitate in the distillation step and reducing an operation load on the distillation column.

In the solvent addition step, the solvent may be added in a stirred tank, or the reaction composition and the solvent may be joined together and then mixed using a static mixer.

<Distillation Step>

In the distillation step according to the present embodiment, the reaction composition is distilled in the distillation column.

The amount of the precipitate in the reaction composition immediately before introduction into the distillation column in the distillation step is preferably 50 mg/L or less, more preferably 30 mg/L or less, and still more preferably 10 mg/L or less. By setting the amount of the precipitate to the range, the production of the precipitate in the distillation step can be more significantly suppressed.

In the distillation step, a fraction containing 99.9% by mass or more of the carbonate having an ethyl group is preferably separated.

The carbonate having an ethyl group that is the target compound in the production method according to the present embodiment is ethyl methyl carbonate or dimethyl carbonate, and preferably ethyl methyl carbonate. These carbonates having an ethyl group are used, for example, as the electrolyte solution for lithium-ion secondary batteries.

As described above, the production method according to the present embodiment enables to provide the method for producing a carbonate having an ethyl group in which the production of the precipitate can be suppressed in the distillation step and the distillation step can be continuously operated for a long term.

[Apparatus for Producing Carbonate Having Ethyl Group]

Hereinafter, the apparatus for producing a carbonate having an ethyl group with which the production method according to the present embodiment is conducted will be described.

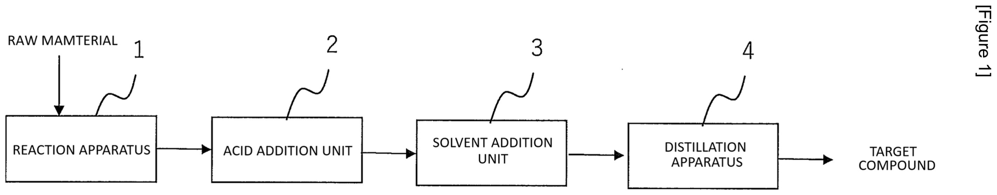

Figure 1 is a block diagram of the apparatus for producing a carbonate having an ethyl group according to the present embodiment. The apparatus for producing a carbonate having an ethyl group according to the present embodiment has a reaction apparatus 1, an acid addition unit2, a solvent addition unit 3, and a distillation apparatus 4. The production apparatus supplies a raw material to the reaction apparatus 1, and sequentially carries out the treatment, so that the reaction step, the acid addition step, the solvent addition step, and the distillation step are carried out in the order presented.

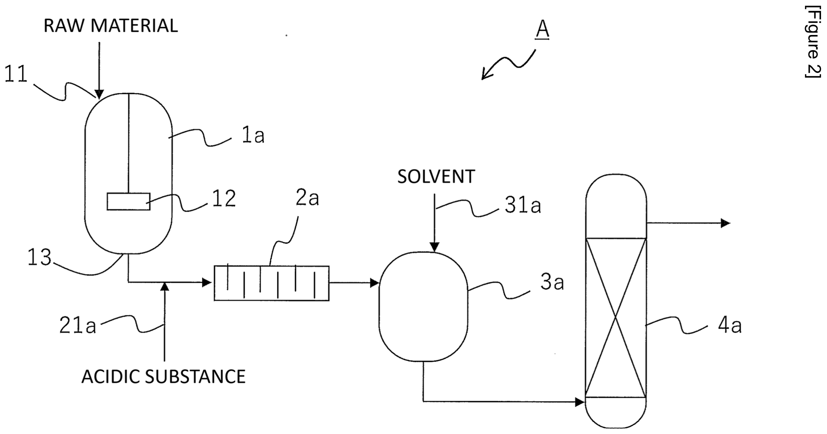

Figure 2 is a schematic configuration diagram of an apparatus A for producing a carbonate having an ethyl group according to the present embodiment. The apparatus A for producing a carbonate having an ethyl group has a stirred tank reactor 1a, an acidic substance supply line 21a, a static mixer 2a, a solvent supply line 31a, a stirred mixing tank 3a, and a distillation column 4a.

A raw material supply unit 11 is provided at the stirred tank reactor 1a, and a raw material is supplied. Here, the raw material is the aforementioned raw material, which contains dimethyl carbonate and ethanol and may contain a catalyst containing an alkali metal compound. The stirred tank reactor 1a has a stirring apparatus 12. An extraction unit 13 is provided at the lower part of the stirred tank reactor 1a, and the reaction composition is extracted from the extraction unit 13.

The reaction composition extracted from the extraction unit 13 is joined together with the acidic substance supplied from the acidic substance supply line 21a, and the alkali metal compound in the reaction composition is deactivated. After being joined together with the acidic substance, the reaction composition is introduced into the static mixer 2a, and the reaction composition is mixed.

Thereafter, the reaction composition is introduced into the stirred mixing tank 3a and mixed with the solvent supplied from the solvent supply line 31a, and the precipitate in the reaction composition is dissolved by the solvent.

The reaction composition is further transferred to the distillation column 4a and purified by distillation to obtain a carbonate having an ethyl group. Since the distillation column 4a can be operated under ordinary pressure and the solvent can be separated from the column bottom, the solvent added from the solvent supply line 31a is preferably a solvent having a higher boiling point than diethyl carbonate under ordinary pressure and having no azeotropic composition, from the viewpoint of not only the quantity of heat, but also the recovery rate of the carbonate having an ethyl group.

As described above, according to the production apparatus A, the raw material supply step, the reaction step, the extraction step, the acid addition step, the solvent addition step, and the distillation step are carried out in the order presented.

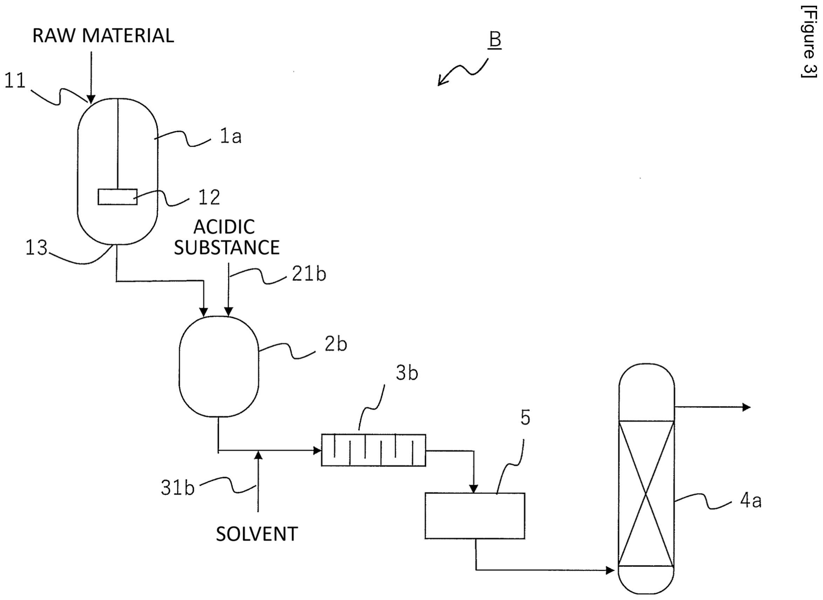

Figure 3 is a schematic configuration diagram of an apparatus B for producing a carbonate having an ethyl group according to the present embodiment. The apparatus B for producing a carbonate having an ethyl group has a stirred tank reactor 1a, an acidic substance supply line 21b, a stirred mixing tank 2b, a solvent supply line 31b, a static mixer 3b, and a distillation column 4a. The portions common to those in the production apparatus A are denoted with the same reference signs, and the detailed description thereof is omitted.

The reaction composition extracted from the extraction unit 13 of the stirred tank reactor 1a is introduced into the stirred mixing tank 2b and mixed with the acidic substance supplied from the acidic substance supply line 21b, and the alkali metal compound in the reaction composition is deactivated.

Thereafter, the reaction composition is joined together with the solvent supplied from the solvent supply line 31b, introduced into the static mixer 3b, and mixed, and the precipitate in the reaction composition is dissolved by the solvent.

The production apparatus B may be equipped with a cushion tank 5. By arranging the cushion tank 5 downstream of the static mixer 3b, the amount of the reaction composition fed to the distillation column 4a can be adjusted.

The reaction composition is further transferred to the distillation column 4a and purified by distillation to obtain a carbonate having an ethyl group.

As described above, according to the production apparatus B, the raw material supply step, the reaction step, the extraction step, the acid addition step, the solvent addition step, and the distillation step are carried out in the order presented.

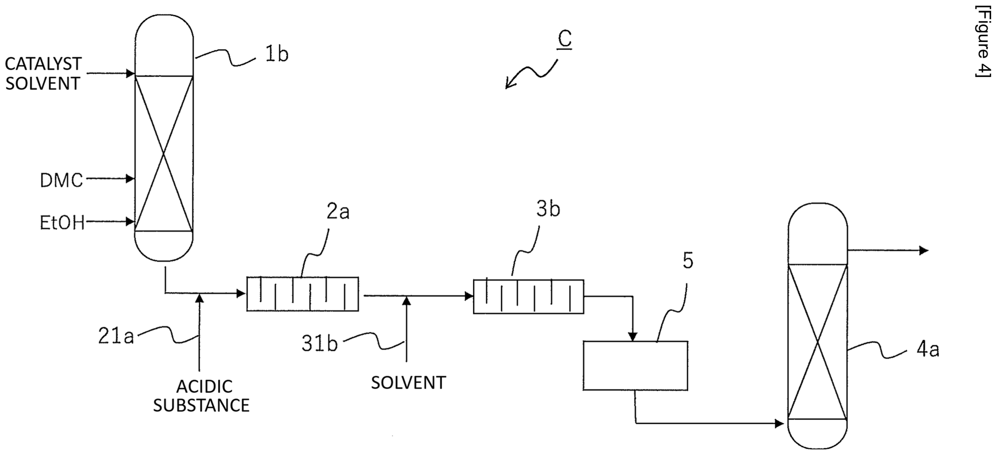

Figure 4 is a schematic configuration diagram of an apparatus C for producing a carbonate having an ethyl group according to the present embodiment. The apparatus C for producing a carbonate having an ethyl group has a reactive distillation column 1b, an acidic substance supply line 21a, a static mixer 2a, a solvent supply line 31b, a static mixer 3b, and a distillation column 4a. The portions common to those in the production apparatus A or the production apparatus B are denoted with the same reference signs, and the detailed description thereof is omitted.

A catalyst solution is introduced from the upper part of the reactive distillation column 1b. As the catalyst solution, a solution obtained by dissolving the aforementioned catalyst in a solvent such as an alcohol is used. From the intermediate part of the reactive distillation column 1b, dimethyl carbonate and ethanol are introduced. Consequently, dimethyl carbonate and ethanol can be subjected to transesterification reaction in the reactive distillation column 1b, and the reaction composition is extracted from the column bottom.

As described above, according to the production apparatus C, the reaction step, the acid addition step, the solvent addition step, and the distillation step are carried out in the order presented.

**Examples**

Hereinafter, the present embodiments will be described in more detail with reference to Examples, but the present invention is not limited to the following Examples.

[Concentration of Insoluble Matter Having Size of 10 $\mu$m or more in Reaction Composition]

10,000 g of a reaction composition was extracted as a sample, and the sample was filtered with a filter made of polytetrafluoroethylene (PTFE) having a pore size of 10 $\mu$m under a reduced pressure of -0.069 MPaG within 90 minutes after sampling. The mass of the filtered substance after completion of the filtration was measured, and the following equation was used as the concentration of insoluble matter in the reaction composition.

(Concentration of insoluble matter having a size of 10 $\mu$m or more in a reaction composition [ppm by mass]) = (mass of filtered substance [g]) ÷ (filtered sample mass [g]) × 1,000,000

[Example 1]

Using the apparatus A for producing a carbonate having an ethyl group having a structure illustrated in Figure 2, carbonates having an ethyl group were produced as follows.

DMC, EtOH, and a 28% by mass solution of sodium methoxide in methanol were respectively supplied to a stirred tank reactor 1a having a capacity of 0.1 m$^3$ at a flow rate of 75 kg/h, 25 kg/h, and 71 g/h, and reacted under the conditions of 70°C and 103 kPaG, and 100 kg/h of the reaction product was extracted from the stirred tank reactor 1a to obtain a reaction composition containing carbonates having an ethyl group. With respect to the amount of each component in the reaction composition, MeOH was 13 kg/h, EtOH was 7 kg/h, DMC was 43 kg/h, EMC was 33 kg/h, and DEC was 4 kg/h. The concentration of insoluble matter having a size of 10 $\mu$m or more in the reaction composition extracted from the stirred tank reactor 1a was 112 ppm by mass.

To the reaction composition obtained by the reaction, 162 g/h of butanoic acid (pKa = 4.8) was added, and the mixture was allowed to pass through a static mixer 2a and supplied to a stirred mixing tank 3a. Simultaneously, 1.1 kg/h of monoethylene glycol (MEG, the solubility of sodium butanoate at 30°C was 15% by mass or more) was supplied into the stirred mixing tank 3a. After stirring, 101.3 kg/h of the product was extracted from the stirred mixing tank 3a, supplied to a distillation column 4a, and distilled. The concentration of insoluble matter having a size of 10 $\mu$m or more in the reaction composition before introduction into the distillation column was 0.3 ppm by mass. In the present operation, no precipitate was present in the liquid extracted from the stirred mixing tank 3a and the column bottom liquid after distillation.

[Example 2]

Using the apparatus B for producing a carbonate having an ethyl group having a structure illustrated in Figure 3, carbonates having an ethyl group were produced as follows.

DMC, EtOH, and a 28% by mass solution of sodium methoxide in methanol were respectively supplied to a stirred tank reactor 1a having a capacity of 3 m$^3$ at a flow rate of 300 kg/h, 300 kg/h, and 0.5 kg/h, and reacted under the conditions of 50°C and 103 kPaG, and 601 kg/h of a reaction composition was extracted from the stirred tank reactor 1a to obtain the reaction composition containing carbonates having an ethyl group. With respect to the amount of each component in the reaction composition, MeOH was 99 kg/h, EtOH was 157 kg/h, DMC was 82 kg/h, EMC was 186 kg/h, and DEC was 77 kg/h. The concentration of insoluble matter having a size of 10 μm or more in the reaction composition extracted from the stirred tank reactor 1a was 388 ppm by mass.

After the reaction composition obtained by the reaction and 2.6 kg/h of propanoic acid (pKa = 4.9) were supplied to a stirred mixing tank 2b, 11 kg/h of triethylene glycol (TEG) was added to 603.7 kg/h of the reaction composition, and the mixture was allowed to pass through a static mixer 3b and then supplied to a cushion tank 5. Thereafter, the reaction composition was supplied from the cushion tank 5 to a distillation column 4a and distilled. The concentration of insoluble matter having a size of 10 μm or more in the reaction composition before introduction into the distillation column 4a was 0.4 ppm by mass. In the present operation, no precipitate was present in the liquid extracted from the cushion tank 5 and the column bottom liquid after distillation.

[Example 3]

Using the apparatus C for producing a carbonate having an ethyl group having a structure illustrated in Figure 4, carbonates having an ethyl group were produced as follows.

DMC, EtOH, and a 28% by mass solution of sodium methoxide in methanol were respectively supplied to a reactive distillation column 1b with trays having a column diameter of 500 mm and a column height of 60 stages at a flow rate of 97 kg/h, 13 kg/h, and 0.16 kg/h. The extraction was carried out at a column bottom extraction rate of 32 kg/h, and the operation was carried out at a reflux ratio of 1.0 and a column top pressure of 103 kPaA to obtain a reaction composition containing carbonates having an ethyl group. With respect to the amount of each component in the reaction composition, DMC was 2 kg/h, EMC was 28 kg/h, and DEC was 2 kg/h. The concentration of insoluble matter having a size of 10 μm or more in the reaction composition extracted from the distillation column reactor was 513 ppm by mass.

After 6.6 kg/h of dichloroacetic acid (pKa = 1.3) was added to the reaction composition obtained by the reaction and the mixture was allowed to pass through a static mixer 2a, 8.8 kg/h of diethylene glycol was added to the reaction composition and the mixture was allowed to pass through a static mixer 3b and then supplied to a cushion tank 5. Thereafter, the reaction composition was supplied from the cushion tank 5 to a distillation column 4a and distilled. The concentration of insoluble matter having a size of 10 μm or more in the reaction composition before introduction into the distillation column was 1.2 ppm by mass. In the present operation, no precipitate was present in the liquid extracted from the cushion tank 5 and the column bottom liquid after distillation. The recovery rate of DEC in the distillation column was 99% or more.

[Example 4]

Using the apparatus A for producing a carbonate having an ethyl group having a structure illustrated in Figure 2, carbonates having an ethyl group were produced as follows.

After 75 kg of DMC, 25 kg of EtOH, and 10 g of a 28% by mass solution of sodium methoxide in methanol were supplied to a stirred tank reactor 1a having a capacity of 0.23 m$^3$ and stirred under the conditions of 70°C and 103 kPaG for 5 h, a reaction composition was extracted from the lower part of the stirred tank reactor 1a at a flow rate of 100 kg/h to obtain the reaction composition containing carbonates having an ethyl group. With respect to the amount of each component in the reaction composition, MeOH was 13 kg/h, EtOH was 6 kg/h, DMC was 44 kg/h, EMC was 33 kg/h, and DEC was 4 kg/h.

After 41 g/h of butanoic acid was added to the reaction composition obtained by the reaction and the mixture was allowed to pass through a static mixer 2a, the reaction composition and 7.3 kg/h of monoethylene glycol were added to a stirred mixing tank 3a, and 107.4 kg/h of the reaction composition was then extracted from the stirred tank, supplied to a distillation column 4a, and distilled. The concentration of insoluble matter having a size of 10 μm or more in the reaction composition before introduction into the distillation column 4a was 0.1 ppm by mass or less, and no precipitate was confirmed. In the present operation, the concentration of insoluble matter in the liquid extracted from the stirred mixing tank 3a and the column bottom liquid after distillation was 0 ppm by mass.

[Example 5]

Using the apparatus B for producing a carbonate having an ethyl group having a structure illustrated in Figure 3, carbonates having an ethyl group were produced as follows.

DMC, EtOH, and a 28% by mass solution of sodium methoxide in methanol were respectively supplied to a stirred tank reactor 1a having a capacity of 3 m$^3$ at a flow rate of 300 kg/h, 300 kg/h, and 0.5 kg/h, and reacted under the conditions of 50°C and 103 kPaG, and 601 kg/h of a reaction composition was extracted from the stirred tank reactor 1a to obtain the reaction composition containing carbonates having an ethyl group. With respect to the amount of each component in the reaction composition, MeOH was 99 kg/h, EtOH was 157 kg/h, DMC was 82 kg/h, EMC was 186 kg/h, and DEC was 77 kg/h. The concentration of insoluble matter having a size of 10 μm or more in the reaction composition extracted from the stirred tank reactor 1a was 388 ppm by mass.

After the reaction composition obtained by the reaction and 2.6 kg/h of propanoic acid (pKa = 4.9) were supplied to a stirred mixing tank 2b, 100 kg/h of 1-butanol was added to 603.7 kg/h of the reaction composition, and the mixture was allowed to pass through a static mixer 3b and then supplied to a cushion tank 5. Thereafter, the reaction composition was supplied from the cushion tank 5 to a distillation column 4a and distilled. The concentration of insoluble matter having a size of 10 μm or more in the reaction composition before introduction into the distillation column 4a was less than 0.1 ppm by mass. In the present operation, no precipitate was present in the liquid extracted from the cushion tank 5 and the column bottom liquid after distillation. The recovery rate of DEC was 40%.

[Comparative Example 1]

Using a production apparatus obtained by removing the acidic substance supply line 21a, the static mixer 2a, the solvent supply line 31a, and the stirred mixing tank 3a from the apparatus A for producing a carbonate having an ethyl group having a structure illustrated in Figure 2, carbonates having an ethyl group were produced as follows.

DMC, EtOH, and a 28% by mass sodium methoxide solution were respectively supplied to a stirred tank reactor 1a having a capacity of 0.1 m$^3$ at a flow rate of 75 kg/h, 25 kg/h, and 71 g/h, and reacted at 70°C and 103 kPaG, and 100 kg/h of a reaction composition was extracted from the stirred tank reactor 1a to obtain carbonates having an ethyl group. The liquid after being extracted from the stirred tank reactor 1a was distilled, and 112 ppm by mass of the precipitate was present in the liquid extracted from the stirred tank reactor 1a and the column bottom liquid after distillation. Due to the blockage of the distillation column, continuous operation for a long period of time was impossible.

[Table 1-1]

| Table 1 (1/2) | | | Example 1 | Example 2 | Example 3 |
|---|---|---|---|---|---|
| Reaction step | Supply flow rate | | DMC 75kg/h EtOH 25kg/h NaOMe solution 71 g/h | DMC 300kg/h EtOH 300kg/h NaOMe solution 0.5kg/h | DMC 97kg/h EtOH 13kg/h NaOMe solution 0.16kg/h |
| | Reactor | | Stirred tank reactor (capacity 0.1m$^3$) | Stirred tank reactor (capacity 3.0m$^3$) | Tray column (column diameter 500 mm/height 60 stages) |
| | Reaction conditions | | 70°C/103kPaA | 50°C/103kPaA | 103kPaA/reflux ratio 1.0 |
| | Extraction flow rate | | 101.3kg/h | 603.7kg/h | BTM extraction: 32 kg/h |
| Acid addition step | Acid | | Butanoic acid | Propionic acid | Dichloroacetic acid |
| | Acid flow rate | | 162g/h | 2.6kg/h | 6.6kg/h |
| | Acid/NaOMe molar ratio | | 1.2 | 1.5 | 1.5 |
| | Mixing means | | Static mixer | Stirred tank | Static mixer |

(continued)

| Table 1 (1/2) | | Example 1 | Example 2 | Example 3 |
|---|---|---|---|---|
| Solvent addition step | Solvent | MEG | TEG | DEG |
| | Solvent flow rate | 1.1kg/h | 11.0kg/h | 8.8kg/h |
| | Mixing step with solvent | Stirred tank | Static mixer | Static mixer |
| Distillation step | | Distillation | Distillation | Distillation |
| Distillation column bottom liquid extraction | | Absence | Absence | Absence |

[Table 1-2]

| Table 1 (2/2) | | Example 4 | Example 5 | Comparative Example 1 |
|---|---|---|---|---|
| Reaction step | Supply flow rate | DMC 75kg EtOH 25kg NaOMe solution 10g | DMC 300kg/h EtOH 300kg/h NaOMe solution 0.5kg/h | DMC 75kg/h EtOH 25kg/h NaOMe solution 71g/h |
| | Reactor | Stirred tank reactor (capacity $0.2m^3$) | Stirred tank reactor (capacity $3.0m^3$) | Stirred tank reactor (capacity $0.1m^3$) |
| | Reaction conditions | 70°C/103kPaA | 50°C/103kPaA | 70°C/103kPaA |
| | Extraction flow rate | 107.4kg/h | 603.7kg/h | 100kg/h |
| Acid addition step | Acid | butanoic acid | Propionic acid | Absence |
| | Acid flow rate | 41g/h | 2.6kg/h | - |
| | Acid/NaOMe molar ratio | 1.2 | 1.5 | - |
| | Mixing means | Static mixer | Stirred tank | - |
| Solvent addition step | Solvent | MEG | 1-Butanol | Absence |
| | Solvent flow rate | 7.3kg/h | 100kg/h | |
| | Mixing step with solvent | Stirred tank | Static mixer | - |
| Distillation step | | Distillation | Distillation | Distillation |
| Distillation column bottom liquid extraction | | Absence | Absence | Presence |

## Industrial Applicability

According to the method for producing a carbonate having an ethyl group of the present invention, substances such as ethyl methyl carbonate and diethyl carbonate used as the electrolyte solution for lithium-ion secondary batteries can be efficiently produced.

## Reference Signs List

1: Reaction apparatus
1a: Stirred tank reactor
11: Raw material supply unit
12: Stirring apparatus
13: Extraction unit
1b: Reactive distillation column
2: Acid addition unit
2a: Static mixer
21a: Acidic substance supply line
3: Solvent addition unit
3a: Stirred mixing tank
31a: Solvent supply line
4: Distillation apparatus
4a: Distillation column

**Claims**

1. A method for producing a carbonate having an ethyl group, the method comprising:

   a reaction step of subjecting dimethyl carbonate and ethanol to transesterification reaction in a reactor in the presence of a catalyst containing an alkali metal compound to obtain a reaction composition;
   a solvent addition step of adding a solvent to the reaction composition; and
   a distillation step of distilling the reaction composition in a distillation column.

2. A method for producing a carbonate having an ethyl group, the method comprising:

   a reaction step of subjecting dimethyl carbonate and ethanol to transesterification reaction in a reactor in the presence of a catalyst containing an alkali metal compound to obtain a reaction composition; and
   a distillation step of distilling the reaction composition in a distillation column,
   wherein the concentration of insoluble matter having a size of 10 $\mu$m or more in the reaction composition immediately before introduction into the distillation column in the distillation step is 50 ppm by mass or less.

3. The method for producing a carbonate having an ethyl group according to claim 1, the method comprising the solvent addition step of adding a solvent to the reaction composition before the distillation step.

4. The method for producing a carbonate having an ethyl group according to claim 1 or 3, wherein in the solvent addition step, insoluble matter in the reaction composition is dissolved.

5. The method for producing a carbonate having an ethyl group according to claim 1 or 3, the method comprising an acid addition step of adding an acidic substance having a pKa of 6.0 or less to the reaction composition before the solvent addition step.

6. The method for producing a carbonate having an ethyl group according to according to claim 5, wherein the acidic substance is at least one selected from the group consisting of carboxylic acid and sulfonic acid.

7. The method for producing a carbonate having an ethyl group according to claim 6, wherein the acidic substance is at least one selected from the group consisting of propanoic acid, butanoic acid, pentanoic acid, hexanoic acid, chloroacetic acid, dichloroacetic acid, trichloroacetic acid, and oleic acid.

8. The method for producing a carbonate having an ethyl group according to claim 5, wherein each of the alkali metal compound and a reaction product of the alkali metal compound and the acidic compound has a solubility of 10% by mass or more at 30°C in the solvent.

9. The method for producing a carbonate having an ethyl group according to claim 1 or 3, wherein the solvent has a higher boiling point than a boiling point of diethyl carbonate under ordinary pressure.

10. The method for producing a carbonate having an ethyl group according to claim 1 or 3, wherein the solvent contains a compound having at least one hydroxyl group.

11. The method for producing a carbonate having an ethyl group according to claim 1 or 3, wherein the solvent contains at least one selected from the group consisting of monoethylene glycol, diethylene glycol, triethylene glycol, 1-hexanol, 2-hexanol, 1-heptanol, and 2-heptanol.

12. The method for producing a carbonate having an ethyl group according to claim 1 or 3, wherein an amount of the solvent added in the solvent addition step is 0.1 parts by mass to 20 parts by mass per 100 parts by mass of the reaction composition.

13. The method for producing a carbonate having an ethyl group according to any one of claims 1 to 3, wherein the alkali metal compound is a sodium-containing compound.

14. The method for producing a carbonate having an ethyl group according to any one of claims 1 to 3, wherein a concentration of the alkali metal compound is 1 ppm by mass to 30,000 ppm by mass based on the total amount of the reaction composition.

15. The method for producing a carbonate having an ethyl group according to any one of claims 1 to 3, wherein the reactor is a stirred reactor or a distillation column.

16. The method for producing a carbonate having an ethyl group according to any one of claims 1 to 3, wherein the distillation step is a step of separating a fraction containing 99.9% by mass or more of the carbonate having an ethyl group.

17. The method for producing a carbonate having an ethyl group according to any one of claims 1 to 3, wherein the carbonate having an ethyl group is ethyl methyl carbonate or dimethyl carbonate.

18. An apparatus for producing a carbonate having an ethyl group, the apparatus comprising:

a reaction apparatus for subjecting dimethyl carbonate and ethanol to transesterification reaction in a reactor in the presence of a catalyst containing an alkali metal compound to obtain a reaction composition;
a solvent addition unit for adding a solvent to the reaction composition; and
a distillation apparatus for distilling the reaction composition.

[Figure 1]

[Figure 2]

EP 4 725 935 A1

17

[Figure 3]

[Figure 4]

## INTERNATIONAL SEARCH REPORT

| International application No. |
|---|
| **PCT/JP2024/020116** |

**A. CLASSIFICATION OF SUBJECT MATTER**

*C07C 68/08*(2006.01)i; *C07B 61/00*(2006.01)i; *C07C 68/06*(2020.01)i; *C07C 69/96*(2006.01)i
FI:     C07C68/08; C07C68/06; C07C69/96 Z; C07B61/00 300

According to International Patent Classification (IPC) or to both national classification and IPC

**B. FIELDS SEARCHED**

Minimum documentation searched (classification system followed by classification symbols)

C07C68/08; C07B61/00; C07C68/06; C07C69/96

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2022/114576 A1 (LOTTE CHEMICAL CORPORATION) 02 June 2022 (2022-06-02) claims, paragraphs [0039]-[0041], examples 1-2 | 2, 13-15, 17 |
| Y | | 16 |
| Y | WO 2014/061678 A1 (UBE INDUSTRIES, LTD.) 24 April 2014 (2014-04-24) claims, paragraph [0158] | 16 |
| A | JP 9-118652 A (MITSUBISHI CHEMICAL CORPORATION) 06 May 1997 (1997-05-06) claims, examples 1-5 | 1-18 |
| A | JP 2010-168365 A (MITSUBISHI CHEMICAL CORPORATION) 05 August 2010 (2010-08-05) claims, examples 1-4 | 1-18 |

☐ Further documents are listed in the continuation of Box C.       ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **05 August 2024** | **20 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/020116**

| Patent document cited in search report | | | Publication date (day/month/year) | Patent family member(s) | | | Publication date (day/month/year) |
|---|---|---|---|---|---|---|---|
| WO | 2022/114576 | A1 | 02 June 2022 | US | 2024/0002330 | A1 | |
| | | | | claims, paragraphs [0037]-[0039], examples 1-2 | | | |
| | | | | EP | 4253364 | A1 | |
| | | | | KR | 10-2022-0073559 | A | |
| | | | | CN | 116745259 | A | |
| | | | | JP | 2023-552122 | A | |
| WO | 2014/061678 | A1 | 24 April 2014 | US | 2015/0291504 | A1 | |
| | | | | claims, paragraph [0112] | | | |
| | | | | CN | 104718183 | A | |
| | | | | KR | 10-2015-0055022 | A | |
| JP | 9-118652 | A | 06 May 1997 | US | 5760273 | A | |
| | | | | KR | 10-1998-0002000 | A | |
| JP | 2010-168365 | A | 05 August 2010 | US | 2011/0313185 | A1 | |
| | | | | WO | 2010/074256 | A1 | |
| | | | | EP | 2380868 | A1 | |
| | | | | KR | 10-2011-0105379 | A | |
| | | | | CN | 102264687 | A | |

Form PCT/ISA/210 (patent family annex) (July 2022)

**REFERENCES CITED IN THE DESCRIPTION**

**Patent documents cited in the description**

- WO 2022114592 A
- WO 2022114576 A